# EUROPEAN PATENT SPECIFICATION

(11) **EP 0 836 842 B1**
(45) Date of publication and mention of the grant of the patent: **04.04.2001**
(21) Application number: 96116588.3
(22) Date of filing: 16.10.1996
(51) Int. Cl.: A61F 13/15, D04H 1/46

(54) **Disposable non-woven cleaning articles**
Wegwerfbare Reinigungs-Gegenstände aus Vliesstoff
Produits de nettoyage non tissé jetables

(43) Date of publication of application: 22.04.1998
(73) Proprietor: THE PROCTER & GAMBLE COMPANY, Cincinnati, Ohio 45202 (US)
(72) Inventor: Heinicke Moore, Katherine Louise, 61462 Königstein (DE); Malmbak, Marianne, 65510 Idstein-Eschenhahn (DE)
(74) Representative: Canonici, Jean-Jacques

(56) References cited:
- FR-A- 2 752 247
- US-A- 4 718 152
- US-A- 5 302 446
- US-A- 5 320 900
- PATENT ABSTRACTS OF JAPAN vol. 016, no. 387 (C-0975), 18 August 1992 & JP 04 126858 A (DAISAN GOUMEIGAISHIYA), 27 April 1992,

## Description

### Field of the invention

This invention relates to disposable non-woven cleaning articles that deliver a superior cleaning performance when correctly oriented on the hand/finger tips of the user. The present invention has particular application to cleaning articles which can be used for cleaning areas of the human person, for example, skin care pads for cosmetic and therapeutic purposes, dry wipes, wet wipes and moist toilet tissue; for cleaning other surfaces, for example, kitchen and bathroom surfaces; and for surfaces which require cleaning in industry, for example, machinery or vehicle surfaces. For simplicity, the ensuing description focuses on cleaning articles for the human skin, especially facial cleansing articles, but what is said must be understood in light of the foregoing comments about the wider applicability of the present invention.

### Background of the invention

Disposable cleaning articles are well known in the art and are available both in the dry and impregnated state. Such articles typically comprise a substrate of non-woven fibres, which is either dry, moistened or wetted by a suitable wet-cleansing solution. Cleaning articles with a dry-based substrate tend to be rubbed against the epidermis, generally in a circular motion, to remove and absorb dirt, grime, dead skin cells, keratin and other debris. Such articles are frequently used as carriers for cleansing products such as oils, cleansing creams and cleansing lotions, which are poured directly onto the articles by the user immediately prior to application. Impregnated cleaning articles are articles saturated in skin cleansing solution for use as wipes and do not generally require as strenuous a rubbing action for efficient removal of skin debris. Both types of cleaning articles are designed to have a high tensile strength, good cleaning performance and softness. Nevertheless, a need exists for a cleaning article that exhibits an improvement in cleaning power, that has a user-friendly shape, that cleans all areas and contours of the face especially around the more delicate regions such as the eyes, that has a low linting level, is void of smearing and is soft and non-abrasive. It has thus surprisingly been found that cleaning articles of an elongated shape and of a particular fibre entanglement orientation can be produced with concomitant anisotropic benefits that are instinctively exploited by the user for a superior cleaning response. It has further been found that the above benefits are particularly enhanced with the selection of specific dimensions for the cleaning articles described herein.

The prior art reveals US 5,062,418, which teaches a soft, bulky, light weight fabric with good absorbency that is suitable for use as a wound dressing. The fabric comprises a hydroentangled substrate and consists of a plurality of parallel spaced apart ribs extending across the width of the fabric in the cross direction. The ribs are interconnected by valleys comprising loose fibre bundles extending in the machine direction. The patent does not disclose a cleaning article with fibre entanglement in a direction parallel to the major axis. Furthermore, the patent does not exploit the shape feature in parallel with the fibre entanglement orientation and the instinctive interaction of the user with the cleaning article in pursuit of superior cleaning performance.

It is further known to construct a face washing puff of cotton pile fabric, disclosed in US 4,932,095, that is in the form of a bag adapted to receive the fingers of the user in its opening. While the bag does offer a directional use, it does not guarantee the most efficient and effective cleaning performance in the lengthwise direction due to finger slippage. Furthermore, the bag does not cover a variety of hand sizes (the bag is typically 11 centimetres length by 8 centimetres in width); it is tiresome and uncomfortable to use since the effectiveness of the cleaning action is dependent on the use of three fingers only; the stitching surrounding the border of the bag may cause irritation and may be slightly abrasive to the skin and delicate areas such as the regions close to the eyes; linting occurs which results in user dissatisfaction; and the bag results in a high utilisation of raw materials, which is not very environmentally friendly.

US-A-5 302 446, issued on April 12^{th} 1994, discloses a two-sided skin care wipe material having two plies of fibrous material bonded together, preferably laminated by ultrasonic bonding.

As a result of highlighting the above prior art attempts, the benefits of the present invention range from a cleaning article of elongated shape which guarantees a superior cleaning performance linked to fibre entanglement orientation; to a cleaning article of such a shape to optimise hand/finger tip coverage; to a cleaning article with an increased active surface area for cleaning; to a cleaning article that is capable of cleaning in a more effective manner all areas of the face especially around the eyes; to an exceptional and effective user/product interaction to ensure a more user-friendly product; to a cleaning article that has a low level of linting and is non-abrasive; to a cleaning article that prevents smearing; to a cleaning article that results in an excellent utilisation of raw materials; and to a cleaning article that leads to a high level of user satisfaction and confidence.

### Summary of the invention

The present invention relates to a disposable non-woven cleaning article with an elongated shape having a major axis and a minor axis, and at least one web of entangled fibres entangled in a direction parallel to the major axis. The cleaning article ranges in size from 30 millimetres to 200 millimetres, preferably 30 to 85 millimetres in the direction of the major axis and from 30 millimetres to 65 millimetres in the direction of the minor axis. In a preferred embodiment, the cleaning article has an oval shape and is particularly suited to facial cleansing. Preferably, the cleaning article herein has dimensions ranging from 40 to 80 millimetres in the direction of the major axis and ranging from 30 to 60 millimetres in the direction of the minor axis.

### Brief description of the drawings

It is believed that the invention will be better understood from the foregoing description in conjunction with the accompanying drawing in which:

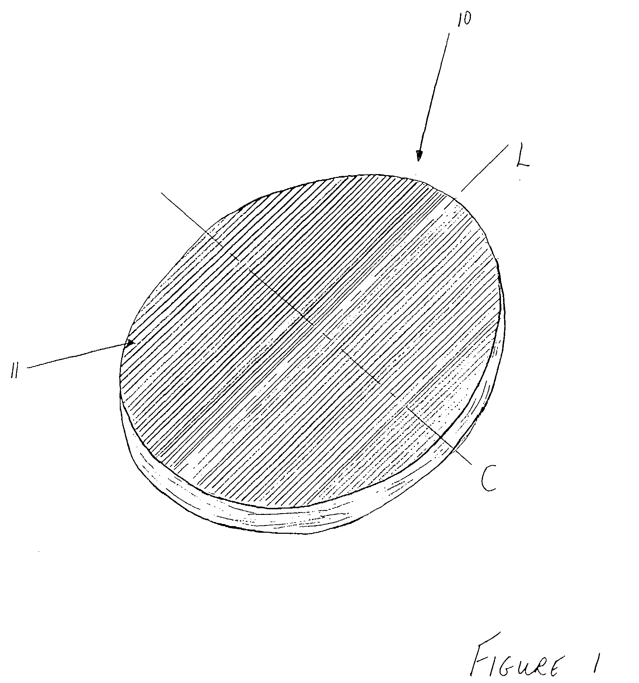

Figure 1 is a plan view of a disposable non-woven cleaning article with an elongated shape, according to the present invention and with dimensions of 64 millimetres for the long axis (L) and 50 millimetres for the minor axis (C).

### Detailed description of the invention

As used herein, the term "disposable" describes absorbent articles that are not intended to be laundered or otherwise restored or reused as an absorbent article (i.e., they are intended to be discarded after a single use and, preferably, to be recycled, composted or otherwise disposed of in an environmentally compatible manner. As used herein, the term "article" refers to a conventional hand-held implement that contains fibres made of cellulosic and/or synthetic material. As meant herein, the term "web" refers to a single layer of fibre material(s). The articles herein may comprise more than one layer, as long as one of the layers is a web according to the present invention and for such a web, a surface for cleaning can be defined. The surface for cleaning is the surface of the web that is brought into closest contact with the desired surface to be cleaned. Additional layers, if present, may be webs of entangled fibres according to the present invention or may be layers of carded or air-laid fibres.

The term "major axis" refers to the longitudinal direction or the y direction or the length and is defined as the direction parallel to the longitudinal centreline of the cleaning article. The term "minor axis" refers to the lateral direction or the x direction or the width and is defined as the direction perpendicular to the longitudinal centreline of the cleaning article.

The present invention relates to a disposable non-woven cleaning article 10. The cleaning article 10 has an elongated shape, which can be defined by a major axis L and a minor axis C. The cleaning article 10 comprises at least one web of entangled fibres 11 with the entangled fibres 11 being entangled in a direction parallel to the major axis L. It has been found that the dimensions of the cleaning article in particular at its ends should be selected in order to provide optimized contact with the fingers of the user. The dimensions of the cleaning article should range from 30 millimetres to 200 millimetres preferably 30 to 85 millimetres in a direction parallel to the major axis L and from 30 millimetres to 65 millimetres in a direction parallel to the minor axis C. In particular, Figure 1 shows a cleaning article 10 which is elongated. It has an arcuate shape. The cleaning article 10 may comprise a variety of shapes. The examples include, but are not limited to, semi-circles, ellipsoids, polygons etc., and any combinations thereof. In a preferred embodiment of the present invention, the cleaning article 10 has an oval shape, which is especially suitable for contact with the human skin, more particularly suitable for facial cleansing. In a more preferred embodiment of the present invention, the cleaning article 10 has dimensions of from 40 to 80 millimetres in a direction parallel to the major axis L and 30 to 60 millimetres in a direction parallel to the minor axis; those dimensions which typically correspond to the finger area of the user have been found to provide the best cleaning results in combination with the entanglement and shape features described herein.

The cleaning article 10 comprises at least one web of entangled fibres 11. The entangled fibres are entangled in a direction parallel to the major axis L of the cleaning article 10. The preferred method of manufacturing the entangled fibres utilises the process of hydroentangling. A hydroentangled web that is strong and durable is produced by traversing material fibres with high energy jet streams of water in order to interlock the fibres. Other high pressure fluids can be used in place of water. Further information pertaining to the hydroentangling process can be found in US A 2,862,551, US A 3,025,585, US A 3,485,706, US A 5,320,900 and EP A 0 418 493. Hydroentangling equipment suitable for use in making the cleaning articles 10 of the present invention is obtainable from ICBT Perfojet, Z.A. Pre-Millet, 38330 Mount Bonnot, France.

It should be mentioned that although hydroentangling is preferred as the method of producing the web, other methods per se in the art can alternatively be used. Needlepunching is an example of a method which can be employed. In this barbed needles are punched through the web, hooking tufts of fibres across it and thereby bonding it in the needlepunched areas. Another method which might be utilised is thermal bonding, in which the fibres are of thermoplastic material, or have an outer layer of thermoplastic material, and are bonded together in discrete spots by heat. A web produced in this way is unlikely to be as soft as one produced by hydroentangling, or needlepunching, and it may have a substantial proportion of completely loose fibres. Yet another method involves wet-laying a mixture of fibres and chemical binder, somewhat in the manner employed, for example, in paper making. Yet an even further method is carding, in which staple fibres are opened, cleaned, aligned and formed into a continuous unbonded web. While hydroentangling, and chemically, thermally, mechanically bonded materials and carded materials are representative of the technologies which can be incorporated in the present invention, these by no means exhaust all of the possibilities. Other technologies such as embossing, creping and aperturing can be incorporated in the manufacture of the cleaning article 10. Such techniques are similarly capable of delivering the desired benefits in accordance with the scope of the present invention.

In a preferred embodiment of the present invention, the cleaning article 10 comprises two webs of entangled fibres 11; the entangled fibres 11 being entangled in a direction parallel to the major axis of the cleaning article 10. The outer surfaces of the webs are referred to as the cleaning surfaces. For the present invention, the cleaning surfaces are located on the top and bottom of the cleaning article 10.

In a preferred embodiment of the present invention, the non-woven entangled web 11 is embossed with an embossing pattern. The embossing pattern typically comprises ridges and valleys, and can be arranged in any shape and in any direction; preferably the embossing pattern should be such that at least a part of the pattern is aligned perpendicular to the major axis L of the cleaning article 10; the dirt, grime and facial debris from the face are thus trapped in the valleys between the ridges in an effective manner and smearing is non-existent. The embossing may be performed using a pair of embossing rollers, which have embossing mounds arranged according to the respective embossing pattern on their external surfaces. The embossing rollers are arranged and controlled in such a way that the embossed mounds are arranged exactly head-to-head in each case but do not touch each other. The present invention is not limited to such a technique and any technique known in the art can be employed to gain the desired embossing pattern.

As mentioned, the web is of entangled fibres 11. Examples of suitable fibre compositions, which can be used to make the cleaning article 10, include purely hydrophilic materials or purely hydrophobic materials or mixtures of a purely hydrophilic fibre material and a purely materials or mixtures of a purely hydrophilic fibre material and a purely hydrophobic material, which can be present in any desired proportions. Hydrophilic materials comprise, for example, cotton, viscose or flax and hydrophobic materials comprise, for example, polyethylene terephthalate or polypropylene. Particularly preferred embodiments include 100 percent cotton; 100 percent viscose; a minimum of 25 percent viscose and the remainder cotton; and a minimum of 25 percent cotton and the remainder viscose; 50 percent viscose and 50 percent polyethylene terephthalate and 50 percent viscose and 50 percent polypropylene. All these percentages are by weight.

The cleaning article 10 can comprise a web(s) that is/are dry or that is/are chemically impregnated in a dry form. Alternatively, the web(s) can be impregnated with a wet-cleansing solution. The wet-cleansing solution can be an aqueous solution or an emulsion in which the continuous phase is aqueous or an oil-based solution in which, for example, the continuous phase is oil.

A suitable method of using a disposable non-woven cleaning article 10 of elongated shape having a major axis and a minor axis will now be described. The cleaning article 10 comprises a web of entangled fibres 11 entangled in a direction parallel to the major axis L. The cleaning article 10 can assume any dimensions although the dimensions specified herein above are preferred. The method comprises the following steps of: positioning the cleaning article 10 on the hand/finger tips of the user; orienting the major axis L of the cleaning article 10 in a direction parallel to the fingers of the user; applying the cleaning article to the surface to be cleaned; and cleaning in a direction parallel to the fingers of the user. The method is particularly suitable if the cleaning article 10 is applied to facial cleansing.

By orienting the major axis L of the cleaning article 10 in a direction parallel to the fingers of the user, the cleaning response is dramatically improved since the cleaning performance is a function of strength and the strength is highest in such a direction. There is also greater efficiency in the cleaning process due to the optimization of the area of coverage of the cleaning article 10 on the fingers/hand of the user for optimised pressure application (from the fingers/hand to the face). In this context, the dimensions of 40 to 80 millimetres in a direction parallel to the major axis, particularly beneficial. For cleaning in the areas close to the eyes, the user holds the cleaning article 10 as before and simply rotates the wrist 90 degrees. This provides for a more natural and more comfortable approach to cleaning and results in a lower level of lining. It is also possible to rotate the cleaning article 90 degrees (in which case, the major axis L is perpendicular to the fingers of the user) and provided that the cleaning motion is still parallel to the fingers of the user, the superior cleaning performance is maintained.

### GLOSSARY

- 10: Cleaning article
- 11: Entangled fibres
- L: Major axis
- C: Minor axis

## Claims

1. Disposable non-woven cleaning article (10) with an elongated shape having a major axis (L) and a minor axis (C), and at least one web of entangled fibres (11),
characterised in that
said entangled fibres (11) are entangled in a direction parallel to said major axis (L), and said cleaning article (10) ranges in size from 30 millimetres to 200 millimetres in the direction of said major axis (L) and from 30 millimetres to 65 millimetres in the direction of said minor axis (C).

2. Cleaning article (10) according to claim 1 wherein said entangled fibres (11) are hydroentangled.

3. Cleaning article (10) according to any of the previous claims wherein said cleaning article (10) comprises two of said webs.

4. Cleaning article (10) according to any of the previous claims wherein said web is embossed with an embossing pattern.

5. Cleaning article (10) according to any of the previous claims wherein said cleaning article (10) has an oval shape.

6. Cleaning article (10) according to any of the previous claims wherein said cleaning article (10) is suitable for facial cleansing.

7. Cleaning article (10) according to any of the previous claims wherein said cleaning article (10) ranges in size from 30 millimetres to 85 millimetres in the direction of said major axis (C).

8. Cleaning article (10) according to claim 7 wherein said cleaning article (10) has dimensions of from 40 to 80 millimetres in a direction parallel to said major axis (L) and 30 to 60 millimetres in a direction parallel to said minor axis (C).

9. Cleaning article (10) according to any of the previous claims wherein said web is dry.

10. Cleaning article (10) according to any of the previous claims wherein said web is impregnated with a wet-cleansing solution.

## Patentansprüche

1. Nach Gebrauch weg zu werfender, reinigender Vliesartikel (10) mit einer länglichen Form mit einer Hauptachse (L) und einer Nebenachse (C) und wenigstens einer Bahn aus verschlungenen Fasern (11),
dadurch gekennzeichnet, daß
die verschlungenen Fasern (11) in einer Richtung parallel zu der Hauptachse (L) verschlungen sind und der reinigende Artikel (10) eine Größe von 30 Millimeter bis 200 Millimeter in Richtung der Hauptachse (L) und von 30 Millimeter bis 65 Millimeter in Richtung der Nebenachse (C) hat.

2. Reinigender Artikel (10) nach Anspruch 1, in welchem die verschlungenen Fasern (11) hydroverschlungen sind.

3. Reinigender Artikel (10) nach einem der vorstehenden Ansprüche, in welchem der reinigende Artikel (10) zwei der Bahnen umfaßt.

4. Reinigender Artikel (10) nach einem der vorstehenden Ansprüche, in welchem die Bahn mit einem Prägemuster geprägt ist.

5. Reinigender Artikel (10) nach einem der vorstehenden Ansprüche, in welchem der reinigende Artikel (10) eine ovale Form hat.

6. Reinigender Artikel (10) nach einem der vorstehenden Ansprüche, in welchem der reinigende Artikel (10) für eine Gesichtsreinigung geeignet ist.

7. Reinigender Artikel (10) nach einem der vorstehenden Ansprüche, in welchem der reinigende Artikel (10) eine Größe von 30 Millimeter bis 85 Millimeter in Richtung der Hauptachse (C) hat.

8. Reinigender Artikel (10) nach Anspruch 7, in welchem der reinigende Artikel (10) Abmessungen von 40 bis 80 Millimeter in einer Richtung parallel zur Hauptachse (L) und 30 bis 60 Millimeter in einer Richtung parallel zur Nebenachse (C) hat.

9. Reinigender Artikel (10) nach einem der vorstehenden Ansprüche, in welchem die Bahn trocken ist.

10. Reinigender Artikel (10) nach einem der vorstehenden Ansprüche, in welchem die Bahn mit einer naßreinigenden Lösung imprägniert ist.

## Revendications

1. Article de nettoyage non tissé jetable (10) de forme allongée présentant un axe principal (L) et un axe secondaire (C), et au moins une nappe de fibres enchevêtrées (11),
caractérisé en ce que
lesdites fibres enchevêtrées (11) sont enchevêtrées dans une direction parallèle audit axe principal (L), et ledit article de nettoyage (10) est compris dans une plage de tailles allant de 30 millimètres à 200 millimètres dans la direction dudit axe principal (L) et de 30 millimètres à 65 millimètres dans la direction dudit axc sccondaire (C).

2. Article de nettoyage (10) selon la revendication 1, dans lequel lesdites fibres enchevêtrées (11) sont hydroenchevêtrées.

3. Article de nettoyage (10) selon l'une quelconque des revendications précédentes, dans lequel ledit article de nettoyage (10) comprend deux desdites nappes.

4. Article de nettoyage (10) selon l'une quelconque des revendications précédentes, dans lequel ladite nappe est gaufrée selon un motif de gaufrage.

5. Article de nettoyage (10) selon l'une quelconque des revendications précédentes, dans lequel ledit article de nettoyage (10) a une forme ovale.

6. Article de nettoyage (10) selon l'une quelconque des revendications précédentes, dans lequel ledit article de nettoyage (10) est approprié pour le nettoyage du visage.

7. Article de nettoyage (10) selon l'une quelconque des revendications précédentes, dans lequel ledit article de nettoyage (10) est compris dans une plage de tailles allant de 30 millimètres à 85 millimètres dans la direction dudit axe principal (L).

8. Article de nettoyage (10) selon la revendication 7, dans lequel l'article de nettoyage (10) a des dimensions comprises entre 40 et 80 millimètres dans une direction parallèle audit axe principal (L) et de 30 à 60 millimètres dans une direction parallèle audit axe secondaire (C).

9. Article de nettoyage (10) selon l'une quelconque des revendications précédentes, dans lequel ladite nappe est sèche.

10. Article de nettoyage (10) selon l'une quelconque des revendications précédentes, dans lequel ladite nappe est imprégnée d'une solution de nettoyage humide.
